# FASCICULE DE BREVET EUROPEEN

(11) **EP 2 238 139 B1**
(45) Date de publication et mention de la délivrance du brevet: **27.09.2017**
(21) Numéro de dépôt: 08869353.6
(22) Date de dépôt: 23.12.2008
(51) Int. Cl.: C07D 473/04, A61K 51/00, C07B 59/00

(54) **PROCÉDÉ DE PRÉPARATION D'UN DÉRIVÉ DE PURINE MARQUÉ, LEDIT DÉRIVÉ ET SES UTILISATIONS**
VERFAHREN ZUR HERSTELLUNG EINES MARKIERTEN PURINDERIVATS, DIESES DERIVAT UND ANWENDUNGEN DAVON
METHOD FOR PREPARING A MARKED PURINE DERIVATIVE, SAID DERIVATIVE AND USES THEREOF

(30) Priorité: 03.01.2008 FR 0850014
(43) Date de publication de la demande: 13.10.2010
(60) Demande divisionnaire: 16152420.2
(73) Titulaire: Commissariat à l'Énergie Atomique et aux Énergies Alternatives, 75015 Paris (FR)
(72) Inventeur: BARRE, Louisa, F-14540 Soliers (FR); MARCHAND, Patrice, F-14220 Fresney le Vieux (FR)
(74) Mandataire: Ilgart, Jean-Christophe
(86) Numéro de dépôt international: PCT/EP2008/068244
(87) Numéro de publication internationale: WO 2009/087066

(56) Documents cités:
- WO-A-2007/063946
- FR-A- 2 841 554
- HORTI A G ET AL: "Synthesis of 2-[<18>F]fluoroadenosine (2-[<18>F]FAD) as potential radiotracer for studying malignancies by PET" JOURNAL OF LABELLED COMPOUNDS AND RADIOPHARMACEUTICALS 200608 GB, vol. 49, no. 9, août 2006 (2006-08), pages 811-815, XP002497780 ISSN: 0362-4803 1099-1344 cité dans la demande
- BRAENDVANG M ET AL: "A novel method for the introduction of fluorine into the purine 2-position: Synthesis of 2-fluoroadenosine and a formal synthesis of the antileukemic drug fludarabine" SYNTHESIS, no. 18, 18 septembre 2006 (2006-09-18), pages 2993-2995, XP002497781 ISSN: 0039-7881 cité dans la demande
- DING Y-S ET AL: "No-Carrier-Added (NCA) {18F}Fluorides via the Nucleophilic Aromatic Substitution of Electron-Rich Aromatic Rings" JOURNAL OF FLUORINE CHEMISTRY, ELSEVIER, NL, vol. 48, no. 2, 1 janvier 1990 (1990-01-01), pages 189-205, XP002215522 ISSN: 0022-1139
- COENEN H H: "Fluorine-18 labeling methods: Features and possibilities of basic reactions" ERNST SCHERING RESEARCH FOUNDATION WORKSHOP, SPRINGER, BERLIN, DE, vol. 62, 7 décembre 2005 (2005-12-07), pages 15-50, XP008097616 ISSN: 0947-6075
- MICHAEL R. KILBOURN: "Nuclear Science Series: Fluorine-18 Labeling of Radiopharmaceuticals" 1990, NATIONAL ACADEMY PRESS , WASHINGTON, D.C. , XP002523586 pages 55-63
- Martine Dhilly ET AL: "2-[18F]Fludarabine, a Novel Positron Emission Tomography (PET) Tracer for Imaging Lymphoma: a Micro-PET Study in Murine Models", Molecular Imaging and Biology, vol. 16, no. 1, 13 July 2013 (2013-07-13), pages 118-126, XP55111853, ISSN: 1536-1632, DOI: 10.1007/s11307-013-0659-2

## Description

### DOMAINE TECHNIQUE

La présente invention appartient au domaine général des composés chimiques marqués et, notamment, des dérivés de purine marqués.

Plus particulièrement, la présente invention concerne un procédé pour marquer au fluor-18 les dérivés de purine. La présente invention concerne également les nouveaux dérivés de purine marqués par le fluor-18 et intermédiaires réactionnels ainsi obtenus et, plus particulièrement, des dérivés de purine marqués en position 2 du noyau aromatique par le fluor-18.

La présente invention concerne également les différentes utilisations de ces nouveaux dérivés de purine et intermédiaires réactionnels marqués par le fluor-18 en imagerie PET, en recherche, en évaluation thérapeutique ou en diagnostic.

### ÉTAT DE LA TECHNIQUE ANTÉRIEURE

La tomographie par émission de positons (ci-après « PET » pour « Positron Emission Tomography »), technique d'imagerie non invasive pour l'évaluation *in vivo* de la distribution des médicaments et leur interaction avec les systèmes cibles biochimiques, est bien adaptée pour l'exploration des différentes fonctions physiologiques et physiopathologiques chez l'homme.

Cet outil est rapidement devenu essentiel en oncologie, neurobiologie et cardiologie. A l'heure actuelle, le défi dans le domaine du PET est de produire et d'offrir, à un coût modéré, des sondes PET utilisables dans de nombreuses applications. Ce but peut être atteint par la mise au point de composés marqués avec un émetteur de positons et produits de façon efficace.

Parmi les sondes PET potentielles, les dérivés de la purine comprenant notamment les nucléosides et les nucléotides font l'objet d'un intérêt croissant, certains de ces dérivés s'étant révélés avoir des activités biologiques dans des domaines variés. De plus, ces dernières années, des efforts croissants ont été déployés en vue de l'élaboration de ligands des récepteurs adénosiniques conduisant au développement d'agents prometteurs basés sur des entités purine ou adénosine (Article de Jacobson K.A. et Gao Z.-G. Nat. Rev. Drug Discovery 2006, 5, 247).

Plusieurs composés portant des groupes purine ou pyrimidine ont déjà été conçus en tant que marqueurs en imagerie PET, et la plupart des stratégies utilisées ont consisté à marquer soit la chaîne latérale, soit le sucre avec un radioisotope. Des purines et adénosines radio-marquées présentant une liaison covalente entre le cycle et l'isotope ont été précédemment décrites ; elles ont notamment été obtenues en utilisant comme radioisotope soit ²H, ³H, ¹⁵N, ¹³C et ¹¹C, soit même ¹⁸F.

Le marquage avec le carbone ¹¹C a largement été étudié, notamment en termes de données biologiques, et breveté (demande internationale WO 03/099342). La [¹¹C]-adénosine mono-phosphate a été obtenue à partir de [¹¹C]-formaldéhyde après 34 minutes et ce, pour un rendement de 2,4%. Cependant, cette méthode présente quelques désavantages : un faible rendement malgré un temps de synthèse correct et la demi-vie courte du carbone ¹¹C (t_{1/2} = 20,4 minutes) qui restreignent ses applications à des examens sur une courte durée. Un autre objet de cette demande concerne la synthèse de 2-[¹⁸F]- fluoroadénosine *via* une réaction de substitution nucléophile par le fluor-18 du sel de diazonium correspondant. Ce procédé inspiré de synthèses classiques (Article de Robins M.J. et Uznanski B., 1981, Can. J. Chem. 59, 2608) est apparu inadapté pour la synthèse de 2-[¹⁸F]-fluoroadénosine et l'inventeur de la demande a récemment rapporté que ce procédé était infaisable (Article de Horti et al., 2006, J. Labelled Compd. Radiopharm., 49, 811).

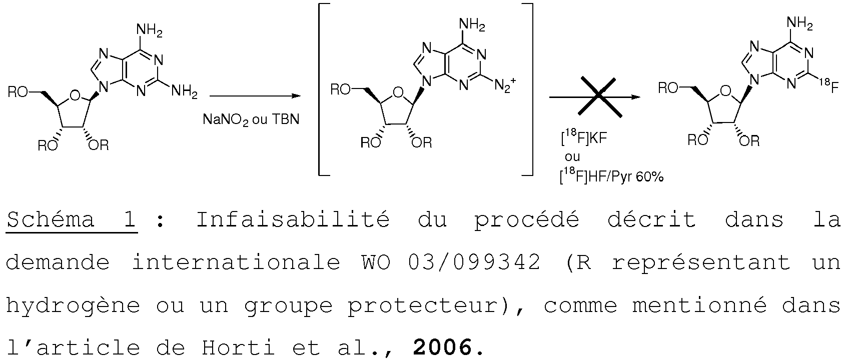

La demande internationale WO 2005/044312 au nom de Bristol-Myers Squibb Company revendique la synthèse de la 2-[¹⁸F]-fluoroadénosine (2- [¹⁸F] FAD) à partir du triflate de 2*-N, N, N-*(triméthylammonium)- adénosine correspondant. Il est bien connu que le triflate de 2*-N,N,N-*(triméthylammonium)-adénosine n'a jamais été isolé voire même jamais observé. Pour l'instant, le matériel de départ est un composé non décrit pour lequel la synthèse a été montrée être infructueuse. Il a été démontré que l'ammonium correspondant ne pouvait être obtenu en utilisant la triméthylamine (Article de Robins M.J. et Uznanski B., 1981, Can. J. Chem. 59, 2601). Les tentatives pour générer l'ammonium correspondant via l'alkylation d'une amine secondaire se sont également soldées par des échecs puisque l'alkylation a toujours lieu au niveau de la position 7 ou 9 du noyau de la purine et non en position 2 (Article de Hocek et al., 2005, Eur. J. Org. Chem. 14, 3026).

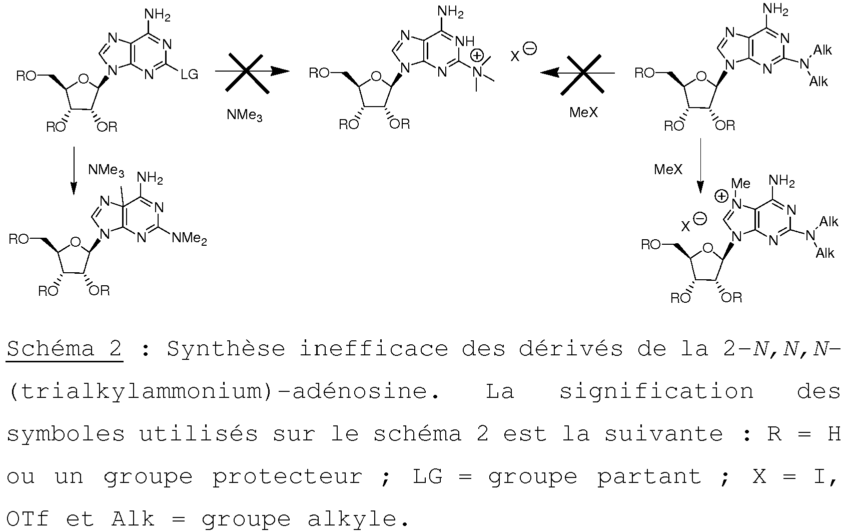

La synthèse de la 2-[¹⁸F]-fluoroadénosine (2-[¹⁸F]FAD) en tant que radiomarqueur potentiel a été réalisée soit à partir de la 2-iodoadénosine correspondante, soit à partir de la 2-fluoroadénosine (substitution isotopique). Dans les deux cas, en partant à partir de nucléosides non protégés, la réaction produit des composés marqués dans des rendements faibles (0,5% et 5% à partir du précurseur iodé ou fluoré, respectivement) et avec des radioactivités spécifiques très faibles (Article de Horti et al., 2006, J. Labelled Compd. Radiopharm. 49, 811).

La demande de brevet EP 1 956 013 au nom de Fujifilm Pharma et Daiichi Sankyo envisage de préparer un composé portant un groupement imidazopyrimidine marqué au fluor 18 à partir d'un composé portant un groupement imidazopyrimidine nitré. D'une part, le schéma réactionnel est purement théorique et sans justification expérimentale. D'autre part, l'éventuelle faisabilité du marquage tel que défini dans ce document sur des groupements imidazopyrimidine ne présage en rien des possibilités et des conditions de marquage d'autres hétérocycles différents tels que des purines.

La préparation de la 2-fluoro-9-benzylpurine marquée au fluor 18 a aussi été décrite par Irie et coll. (T. Irie, K. Fukushi, O. Isnoue, T. Yamasaki, T. Ido, T. Nozaki Int. J. Appl. Radiat. Isot., 33, 1982, 633-636) à partir de fluorure d'argent marqué au fluor-18. La méthode décrite nécessite l'utilisation de fluorure d'argent en excès et ne conduit qu'à un rendement de 5,4-6,7%. Cette méthode peu efficace s'avère inapplicable pour le développement d'un radio-pharmaceutique.

Le marquage en position 6 a également été décrit en utilisant l'ion fluorure [¹⁸F]- via un déplacement nucléophile du sel de triméthylammonium correspondant. Contrairement à la position 2, la position 6 de la purine peut être fonctionnalisée *via* son ammonium (Article de Irie, et al., 1982, Int. J. Appl. Radiat. Isot. 33(6), 445).

Malgré l'intérêt croissant de ces puissants agents en imagerie PET, aucun procédé efficace pour marquer une molécule comprenant un noyau purine en position 2 du noyau aromatique en utilisant du fluor [¹⁸F] n'a été décrite jusqu'à présent.

De plus, l'introduction d'un atome d'halogène en position 2 de l'adénosine (et des dérivés à base d'adénine) est connue pour augmenter la demi-vie biologique de la molécule et, par conséquent, ses effets principalement via l'inhibition de l'activité enzymatique de l'adénosine désaminase. Ainsi, considérant le besoin d'accéder à de nouveaux agents PET, le potentiel des nucléosides marqués pour le diagnostic de cancers ou l'évaluation de drogues et d'efficacité de traitement et l'intérêt croissant des récepteurs adénosiniques, il existe un besoin évident pour une stratégie plus efficace de radiomarquage des purines et, en particulier, en position 2 du noyau aromatique. Par conséquent, une radio-synthèse efficace de dérivés de 2-[¹⁸F]fluoro-purine serait un progrés significatif dans le domaine de l'imagerie PET.

### EXPOSÉ DE L'INVENTION

La présente invention permet de résoudre les problèmes techniques précédemment décrits et d'apporter une solution au besoin présenté ci-dessus en proposant une méthode originale pour marquer les purines et dérivés de purine (tels que les nucléosides) en utilisant un atome de fluor [¹⁸F].

Dans ce qui suit et ce qui précède, le système de numérotation des atomes généralement accepté pour le noyau purine et représenté ci-après est utilisé :

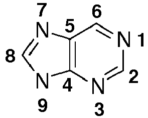

La présente invention propose un moyen facile et grandement efficace pour préparer des dérivés de 2-[¹⁸F] fluoro-purines protégées ou non. Le procédé objet de l'invention est particulièrement efficace dans le cas des dérivés de l'adénosine et de l'adénine tels que, à titre d'exemples non limitatifs, les nucléosides.

Le procédé de la présente invention est remarquable de par le fait que ce procédé fiable peut facilement s'appliquer à différents dérivés de 2-nitro-purines et, grâce à la simplicité et l'efficacité du procédé de l'invention, une procédure appropriée et complètement automatisée pourrait être développée.

La présente invention concerne un procédé de préparation d'un dérivé de 2-fluoro-purine marqué avec le radioisotope ¹⁸F comprenant une étape de fluoration d'un dérivé de 2-nitro-purine protégé de formule (1c) tel que défini ci-après.

Plus particulièrement, l'étape de fluoration du procédé selon la présente invention consiste à faire réagir un dérivé de 2-nitro-purine protégé de formule (1c) tel que défini ci-après avec une source d'ions fluorure F- marqués au [¹⁸F] pour obtenir le dérivé de 2-[¹⁸F] fluoro-purine.

Ainsi, la présente invention concerne un procédé de préparation d'un dérivé de 2-fluoro-purine marqué avec le radioisotope ¹⁸F, comprenant une étape de fluoration consistant à faire réagir un dérivé de 2-nitro-purine protégé avec une source d'ions fluorure F- marqués au [¹⁸F] suivie d'une étape de déprotection, pour obtenir le dérivé de 2-[¹⁸F]fluoro-purine.

Fait intéressant, le procédé selon l'invention peut être mis en oeuvre avec un très large défaut de fluor vis-à-vis du dérivé de 2-nitro-purine, ce qui rend ce procédé utilisable dans la chimie du fluor-18. Avantageusement, le fluor peut être utilisé dans des quantités inférieures ou égales à 10⁻¹, voire inférieures ou égales à 10⁻² équivalent par rapport au dérivé de 2-nitro-purine.

On entend par « dérivé de 2-fluoro-purine marqué avec le radioisotope ¹⁸F » un composé comprenant un noyau purine et de formule (Ia) 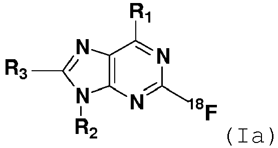 dans laquelle
R₁ représente H, un groupe alkyle éventuellement substitué, un groupe aryle éventuellement substitué, un groupe NR₄R₅,
R₂ représente H, un groupe alkyle éventuellement substitué, un groupe aryle éventuellement substitué, un groupe furanose éventuellement substitué ou un groupe pyranose éventuellement substitué,
R₃ représente H, un groupe alkyle éventuellement substitué, un groupe aryle éventuellement substitué, un halogène, un groupe -OR₈ ou un groupe -SR₈, avec R₈ représentant H, un groupe alkyle éventuellement substitué ou un groupe aryle éventuellement substitué,
R₄ et R₅ représentent indépendamment H, un groupe électroattracteur, un groupe alkyle éventuellement substitué, un groupe aryle éventuellement substitué, un groupe acyle éventuellement substitué, un groupe sulfinyle éventuellement substitué ou un groupe sulfonyle éventuellement substitué.

Les dérivés de 2-fluoro-purine marqués avec le radioisotope ¹⁸F préparés par le procédé de la présente invention sont des composés de formule (Ia) dans laquelle R₁ représente un groupe NH₂.

Des groupes furanose avantageusement mis en oeuvre dans le cadre de la présente invention sont les groupes ribofuranose et arabinofuranose.

Dans le cadre de la présente invention, on entend par « groupe alkyle » un groupe alkyle linéaire, ramifié ou cyclique, éventuellement substitué, de 1 à 20 atomes de carbone, notamment de 1 à 10 atomes de carbone, en particulier, de 1 à 8 atomes de carbone, tout particulièrement, de 1 à 6 atomes de carbone.

Dans le cadre de la présente invention, on entend par « groupe aryle » un groupe aromatique mono- ou polycyclique, éventuellement substitué, ayant de 6 à 20 atomes de carbone, notamment de 6 à 14 atomes de carbone, en particulier, de 6 à 8 atomes de carbone. A titre d'exemples de groupe aryle selon l'invention, on peut citer les groupes phényle, napht-1-yle, napht-2-yle, anthracen-9-yl, 1,2,3,4-tétrahydronapht-5-yle et 1,2,3,4-tétrahydronapht-6-yle.

Dans le cadre de la présente invention, on entend par « groupe acyle » un groupe acyle linéaire ou ramifié, éventuellement substitué, de 1 à 10 atomes de carbone, en particulier, de 1 à 8 atomes de carbone, tout particulièrement, de 1 à 6 atomes de carbone.

Dans le cadre de la présente invention, on entend par « groupe électroattracteur », un groupe choisi parmi les groupes NO₂ et nitrile, des dérivés d'imides et d'imines, un groupe carbonyle -C(=O)-R, un groupe -OC(=O)-R dans lesquels R est un atome d'hydrogène, un groupe OH, un groupe alkyle, un groupe alcoxy, un groupe aryle éventuellement substitués.

Dans le cadre de la présente invention, on entend par « groupe sulfinyle » un groupe de formule RSO-, R étant un groupe alkyle ou aryle tel que précédemment défini.

Dans le cadre de la présente invention, on entend par « groupe sulfonyle » un groupe de formule RSO₂-, R étant un groupe alkyle ou aryle tel que précédemment défini.

Dans le cadre de la présente invention, on entend par « éventuellement substitué » un radical substitué par un ou plusieurs groupes choisis parmi : un groupe alkyle, un groupe alcoxy, un halogène, un hydroxy, un cyano, un trifluorométhyle, un nitro ou un groupe protecteur.

Dans le cadre de la présente invention, on entend par « groupe alcoxy » un atome d'oxygène substitué par un alkyle tel que précédemment défini.

Dans le cadre de la présente invention, on entend par « halogène » un fluor, chlore, brome ou iode.

Dans l'étape de fluoration du procédé selon l'invention, la source d'ions fluorure marqués au ¹⁸F comprend lesdits ions fluorure et un contre-ion, choisi parmi les cations de grande taille et les cations de petite taille.

Un cation de grande taille avantageusement mis en oeuvre dans le cadre de la présente invention est le tétrabutylammonium (Bu₄N⁺). Le fluorure de tétrabutylammonium marqué avec le radioisotope ¹⁸F (Bu₄N[¹⁸F]F) est habituellement préparé en utilisant du Bu₄NOH ou du Bu₄NHCO₃.

A titre d'exemples non limitatifs de cations de petite taille, on peut citer le potassium, le sodium et le lithium. Lesdits cations de petite taille peuvent avantageusement être piégés, stabilisés, par exemple, par un cryptand ou un éther couronne, etc..., ledit cryptand ou éther couronne étant adapté au cation de petite taille mis en oeuvre. Un exemple de cryptand susceptible d'être mis en oeuvre dans le cadre de la présente invention est le produit KRYPTOFIX^{®} K₂₂₂ : (4,7,13,16,21,24-hexaoxa-1,10-diaza bicyclo[8.8.8]hexacosane) qui piège, par exemple, l'ion potassium.

Le contre-ion ou cation peut être amené sous la forme d'un sel quelconque, par exemple, il peut s'agir de K₂CO₃, de K₂SO₄ ou d'oxalate de potassium dans le cas du potassium. Il convient de remarquer que, le sel et, en particulier, le K₂CO₃ devrait avantageusement être utilisé, lors de l'étape de fluoration, à une quantité inférieure ou égale à 1 mg/mg de dérivé de 2-nitro-purine, notamment une quantité inférieure ou égale à 0,6 mg/mg de dérivé de 2-nitro-purine et, en particulier, une quantité inférieure ou égale à 0,1 mg/mg de dérivé de 2-nitro-purine.

L'étape de fluoration du procédé selon l'invention est réalisée dans un solvant, qui est le CH₃CN.

L'étape de fluoration du procédé selon l'invention peut être réalisée dans des conditions connues de l'homme du métier, avec un chauffage à une température comprise entre 55 et 60 °C. Dans cette fourchette de température, le solvant CH₃CN a donné les meilleurs résultats.

L'étape de fluoration du procédé selon l'invention est effectuée sur une durée comprise entre 1 et 30 min, notamment, entre 2 et 20 min, en particulier, entre 5 et 10 min et, tout particulièrement, sur une durée de 8 min.

Dans l'invention, le procédé de préparation d'un dérivé de 2-fluoro-purine marqué avec le radioisotope ¹⁸F comprend les étapes successives suivantes consistant à :
a) faire réagir un dérivé de 2-nitro-purine protégé avec une source d'ions fluorure F- marqués au [¹⁸F] pour obtenir un dérivé de 2-[¹⁸F]fluoro-purine protégé et éventuellement un dérivé de 2-[¹⁸F]fluoro-purine partiellement déprotégé,
b) déprotéger ledit dérivé de 2-[¹⁸F]fluoro-purine protégé et ledit dérivé de 2-[¹⁸F]fluoro-purine partiellement déprotégé éventuellement obtenu pour obtenir le dérivé de 2-[¹⁸F]fluoro-purine.

L'utilisation d'un groupe protecteur approprié notamment sur le noyau purine et, en particulier, deux groupes protecteurs sur l'azote en position 6 du noyau purine augmente considérablement le rendement des composés marqués et réduit le temps de synthèse. Cette forme de mise en oeuvre particulière peut être représentée de la façon schématique suivante (Schéma 3) :

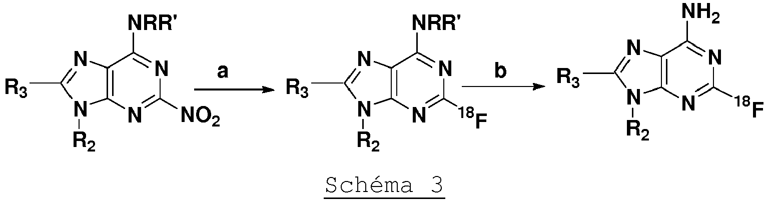

Le dérivé de 2-nitro-purine protégé mis en oeuvre dans le cadre de la présente invention est de formule (Ic) 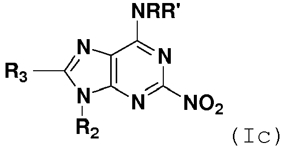 dans laquelle R et R' sont des groupes protecteurs identiques ou différents et R₂ et R₃ sont tels que précédemment définis.

Un groupe protecteur mis en oeuvre dans le cadre de la présente invention est choisi parmi un groupe alkyle éventuellement substitué, un groupe acyle éventuellement substitué, un groupe aryle éventuellement substitué, un groupe benzyle éventuellement substitué, un groupe benzoyle éventuellement substitué, un groupe électroattracteur, un groupe sulfinyle éventuellement substitué, un groupe sulfonyle éventuellement substitué, un groupe trityle, un groupe silyle, un groupe tertiobutoxycarbonyle (BOC), un groupe fluorénylméthoxy carbonyle (FMOC) et un dérivé d'imide ou un dérivé d'imine.

Il convient de remarquer que les composés de formule (Ic) mis en oeuvre dans le cadre de la présente invention comprennent au moins deux groupes protecteurs que sont les groupes protecteurs R et R' sur l'azote en position 6 du noyau purine mais peuvent comprendre d'autres groupes protecteurs. Ces autres groupes protecteurs sont utilisés pour protéger les groupes des composés de formule (Ic) susceptibles d'interagir avec la source d'ions fluorure lors de l'étape de fluoration (a). Cet aspect est notamment exemplifié, dans la partie expérimentale ci-après, avec la 2-nitro-pentabenzoyladénosine et le précurseur 7. En effet, ces deux composés présentent trois groupes protecteurs en plus des deux groupes protecteurs R et R', ces trois groupes additionnels protégeant les trois groupes hydroxyles du ribofuranose.

De tels composés de formule (Ic) sont facilement accessibles à l'homme du métier. En effet, la partie expérimentale ci-après propose plusieurs procédés de synthèse permettant d'obtenir des composés de formule (Ic) et, plus particulièrement, la 2-nitro-pentabenzoyladénosine et le précurseur 7. Il est facile, pour l'homme du métier, de préparer, à partir de cet enseignement, l'ensemble des composés de formule (Ic).

L'étape (a) du procédé selon l'invention correspond à l'étape de fluoration telle que précédemment définie. Par conséquent, tout ce qui a été précédemment développé pour caractériser cette étape de fluoration et notamment concernant l'ion fluorure, le contre-ion, le solvant, la température et la durée de l'étape de fluoration s'applique également à l'étape (a) du procédé selon l'invention.

Le dérivé de 2-[¹⁸F]fluoro-purine protégé obtenu après l'étape (a) du procédé selon l'invention est de formule (Id) 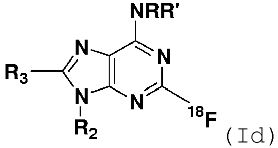 avec R₂, R₃, R et R' tels que précédemment définis.

En outre, en fonction des conditions expérimentales mises en oeuvre lors de cette étape (a) de fluoration, le dérivé de 2-[¹⁸F]fluoro-purine protégé de formule (Id) pourra être obtenu, suite à l'étape (a) du procédé selon l'invention, en mélange avec un dérivé de 2-[¹⁸F]fluoro-purine partiellement déprotégé. Le dérivé de 2-[¹⁸F]fluoro-purine partiellement déprotégé peut représenter de 0 à 80 % du mélange (dérivé de 2-[¹⁸F] fluoro-purine protégé + dérivé de 2-[¹⁸F]fluoro-purine partiellement déprotégé) obtenu en fin d'étape de fluoration (a). Le dérivé de 2-[¹⁸F]fluoro-purine partiellement déprotégé est de formule (Ie) 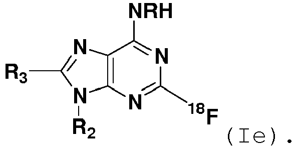 avec R₂, R₃ et R tels que précédemment définis.

Les travaux des inventeurs ont notamment montré que, lorsque du K₂SO₄ est utilisé comme source de cations lors de l'étape de fluoration (a), le dérivé de formule (Ie) est majoritairement obtenu.

Par conséquent, la présente description concerne également l'utilisation de K₂SO₄ pour préparer, par fluoration directe, un dérivé de 2-[¹⁸F]fluoro-purine partiellement déprotégé de formule (Ie) tel que précédemment défini à partir d'un dérivé de 2-nitro-purine protégé de formule (Ic) tel que précédemmment défini.

La mise en oeuvre de l'étape (b) peut présenter deux variantes.

Dans une première variante mise en oeuvre dans le procédé selon l'invention, cette étape (b) de déprotection est effectuée en une seule étape permettant de passer, sans intermédiaire, du composé de formule (Id) éventuellement mélangé avec le composé partiellement déprotégé de formule (Ie) au dérivé de 2-[¹⁸F]fluoro-purine de formule (If) 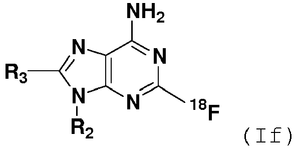 avec R₂ et R₃ tels que précédemment définis.

Dans cette variante, l'élimination des groupes protecteurs de la fonction amine en position 6 du noyau purine, i.e. la déprotection, pour donner le composé de formule (If) dans lequel le groupe amino est libre, peut être réalisée par tout procédé de déprotection connu de l'homme du métier. Ce dernier saura, en fonction du groupe ou des groupes protecteurs mis en oeuvre, choisir le procédé de déprotection le mieux adapté.

Dans la présente invention, l'étape (b) de déprotection est réalisée en faisant réagir le dérivé de 2-[¹⁸F]fluoro-purine protégé de formule (Id) et le dérivé de 2-[¹⁸F]fluoro-purine partiellement déprotégé de formule (Ie) éventuellement présent avec un mélange d'alcool et d'ammoniac aqueux. Ainsi, l'étape (b) de déprotection est réalisée en faisant réagir le dérivé de 2-[¹⁸F]fluoro-purine protégé et le dérivé de 2-[¹⁸F]fluoro-purine partiellement déprotégé éventuellement présent avec un alcool tel que du méthanol, suivi d'ammoniac aqueux puis en chauffant à une température comprise entre 50 et 90 °C et notamment 70 °C pendant une durée comprise entre 5 et 45 min, notamment entre 10 et 30 min et, en particulier pendant 20 min. Le rapport mélange (alcool + ammoniac aqueux)/eau (v/v) est compris entre 10 et 50 %, notamment 20 et 40 % et, en particulier, est de 28 % Dans le mélange (alcool + ammoniac aqueux), les proportions alcool/ammoniac aqueux exprimées en volume sont comprises entre 5/1 et 1/5, notamment 2/1 et 1/2 et, en particulier, 1/1. Lorsque le composé de formule (Id) est la 2-fluoro-pentabenzoyladénosine marquée avec le radioisotope ¹⁸F, de meilleurs résultats sont habituellement obtenus si la température est maintenue inférieure à 80 °C et ce, pour un temps de réaction de 20 min.

Dans une seconde variante ne faisant pas partie de la présente invention, cette étape (b) de déprotection est réalisée en deux sous-étapes (b') et (b").

Dans cette variante, l'étape (b') consiste à faire réagir le dérivé de 2-fluoro-purine marqué avec le radioisotope ¹⁸F et protégé de formule (Id) obtenu après l'étape (a) de fluoration avec un composé nucléophile pour obtenir un dérivé de 2-fluoro-purine marqué avec le radioisotope ¹⁸F mono-hydrolysé (ou mono-déprotégé) de formule (Ie) tel que précédemment défini.

Le composé nucléophile avantageusement mis en oeuvre lors de la sous-étape (b') est choisi parmi les composés comprenant au moins un atome d'azote porteur d'un doublet libre inclus dans un cycle saturé, insaturé ou aromatique, ledit cycle comprenant de préférence de 3 à 8 atomes ; les amines primaires ou secondaires telles que la 2-phényl-éthylamine ; un dérivé d'hydrazine ou d'hydrazone ; un amide ; un sulfonamide ; un dérivé de l'urée ; un dérivé hétérocyclique de préférence azoté et/ou soufré ; un alcool et un dérivé de phénol. Les articles de Nowak et al., J. Org. Chem. 70, 2005, 7455-7458 et de Ishidoet al., J. Chem. Soc., Perkin Trans. 1, 1977, 657-660 décrivent certains des composés nucléophiles cités ci-dessus et leurs utilisations.

La sous-étape (b') est généralement réalisée dans un solvant, qui peut être tout solvant conventionnel connu de l'homme du métier. A titre d'exemples non exhaustifs de tels solvants, on peut citer le DMSO, le DMF, le CH₃CN et le THF. Avantageusement, le solvant utilisé lors de la sous-étape (b') est identique au solvant mis en oeuvre lors de l'étape (a).

La sous-étape (b') peut être réalisée dans des conditions connues de l'homme du métier, avec un chauffage généralement à une température comprise entre 40 à 100 °C, notamment comprise entre 50 et 80 °C, en particulier de l'ordre de 60 °C.

La sous-étape (b') est effectuée sur une durée comprise entre 1 et 45 min, notamment, entre 2 et 30 min, en particulier, entre 5 et 20 min et, tout particulièrement, sur une durée de 10 min.

Aucune modification n'est à apporter aux conditions de la sous-étape (b') du procédé telle que définie ci-dessus dans le cas où du composé de formule (Ie) est déjà présent en mélange avec le composé de formule (Id) suite à l'étape (a) de fluoration.

Dans cette seconde variante, la sous-étape (b") est une déprotection visant à éliminer le (ou les) groupe(s) protecteur(s) restant(s). Cette sous-étape (b") peut être réalisée par tout procédé de déprotection connu de l'homme du métier. Ce dernier saura, en fonction du groupe (ou des) groupe(s) protecteur(s) restant(s), choisir le procédé de déprotection le mieux adapté. Il convient toutefois de souligner que, compte-tenu de l'étape (b') mise en oeuvre au préalable, les conditions lors de la sous-étape de déprotection (b") peuvent être plus drastiques que les conditions utilisées lors de la déprotection selon la première alternative (étape (b)), notamment en termes de température de réaction.

A titre d'exemples et de façon non limitative, lorsque les groupes protecteurs sont des groupes benzoyles, la sous-étape (b") peut être réalisée en faisant réagir le composé de formule (Ie) avec un alcool tel que du méthanol, suivi d'ammoniac aqueux puis en chauffant à une température comprise entre 50 et 110 °C, notamment comprise entre 70 et 90 °C et, en particulier, comprise entre 80 et 85 °C pendant une durée comprise entre 5 et 45 min, notamment entre 10 et 30 min et, en particulier pendant 20 min.

Les étapes de déprotection (b) et (b") sont suivies d'une étape d'hydrolyse. Toute technique d'hydrolyse connue de l'homme du métier est susceptible d'être mise en oeuvre dans le cadre de la présente invention. A titre d'exemples non limitatifs, on peut citer une hydrolyse réalisée avec une solution aqueuse d'acide acétique ou une élution via une résine acide.

La purification du dérivé de 2-fluoro-purine marqué avec le radioisotope ¹⁸F suite au procédé de l'invention peut être effectuée, si nécessaire, par toute technique de purification connue de l'homme du métier. A titre d'exemples non limitatifs, on peut citer une chromatographie, une chromatographie flash, une chromatotographie flash sur gel de silice, une HPLC semi-préparative, etc...

Le procédé selon l'invention est simple, fiable, facile à mettre en oeuvre et peut être aisément robotisé.

La substitution nucléophile d'un dérivé de 2-nitro-purine qui peut être, si nécessaire, protégé, par des ions fluorure sous forme de [¹⁸F]-KF ou [¹⁸F]-Bu₄NF conduit, après déprotection et purification au dérivé de 2-fluoro-purine radiomarqué attendu à un rendement global élevé.

L'incorporation de l'halogène fluor-18 permettant d'obtenir des dérivés de 2-nitro-purine protégés est réalisée de manière extrêmement efficace avec un fort rendement, par exemple de l'ordre de 70 à 100 %, notamment de 80 à 100 % et plus particulièrement de 90 à 98 %. L'invention couvre aussi la possibilité d'obtenir, de façon sélective, avec un rendement élevé, un dérivé de 2-fluoro-purine radiomarqué déprotégé.

Le rendement final de l'ensemble du procédé pour un produit purifié est extrêmement élevé, par exemple de l'ordre de 40-60 % et ce quelle que soit la variante de procédé mise en oeuvre. Il convient de souligner que la présence ou non d'un dérivé de 2-[¹⁸F]fluoro-purine partiellement déprotégé de formule (Ie) suite à l'étape (a) de fluoration n'affecte pas le rendement final du procédé selon l'invention.

Des exemples d'application décrivent la synthèse de la [¹⁸F]-2-fluoroadénosine et de la [¹⁸F]-fludarabine *via* une fluoration avec [¹⁸F] et une déprotection. En comparant à d'autres procédés, les rendements améliorés sont obtenus à partir d'intermédiaires facilement disponibles dans un temps de réaction court, incluant la purification, ce qui rend le procédé selon l'invention compatible avec une étude PET.

En effet, La durée globale du procédé selon l'invention est faible : à titre d'exemple, elle est généralement de 60 à 120 minutes, de préférence 75 à 85 minutes.

La présente description fournit également un procédé de préparation d'un dérivé de 2-fluoro-purine marqué avec le radioisotope ¹⁸F mono-hydrolysé (ou mono-déprotégé) de formule (Ie) telle que précédemment définie. Ledit procédé consiste à faire réagir un dérivé de 2-fluoro-purine marqué avec le radioisotope ¹⁸F et protégé de formule (Id) telle que précédemment définie avec un composé nucléophile tel que précédemment défini, et ce dans les conditions précédemment décrites pour la sous-étape (b').

La présente description fournit également un composé susceptible d'être préparé par un procédé selon la présente invention ou d'être obtenu au cours d'un procédé selon la présente invention (intermédiaire réactionnel).

De façon avantageuse, un tel composé est un composé de formule (A) 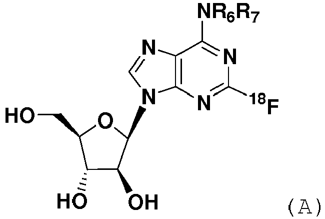 dans laquelle R₆ et R₇ sont indépendamment H ou un groupe protecteur ,
ou un sel de celui-ci.

Parmi ces composés, les composés suivants sont particulièrement préférées :
- la fludarabine marquée avec le radioisotope ¹⁸F de formule (B) ou un de ses sels

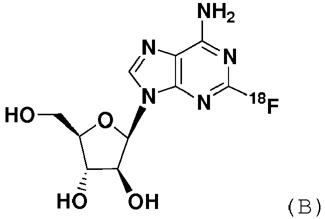

Par « sel », on entend, dans le cadre de la présente invention, les sels d'addition d'acides et les sels d'addition de bases. De tels sels peuvent être formés par des moyens conventionnels, par exemple par réaction d'une forme d'acide libre ou d'une forme de base libre d'un composé de l'invention avec un ou plusieurs équivalents d'un acide ou d'une base approprié, facultativement dans un solvant, ou dans un milieu dans lequel le sel est insoluble, puis par extraction dudit solvant, ou dudit milieu, en utilisant des techniques standard (par exemple sous vide ou par lyophilisation). Les sels peuvent également être préparés en remplaçant un contre-ion d'un composé de l'invention sous la forme d'un sel par un autre contre-ion, par exemple en utilisant une résine échangeuse d'ions appropriée.

Notamment dans le but d'une administration au corps humain ou animal, les sels de ces composés sont avantageusement des sels pharmaceutiquement acceptables.

En particulier, lorsque ces composés se présentent sous forme d'un sel, il s'agit d'un sel d'un métal alcalin, en particulier, un sel de sodium ou de potassium, ou d'un sel d'un métal alcalino-terreux, en particulier le magnésium ou le calcium, ou encore d'un sel avec une amine organique, plus particulièrement, avec un acide aminé tel que l'arginine ou la lysine.

Lorsque ces composés possèdent une fonction amine et se présentent sous la forme d'un sel de cette amine, il s'agit d'un sel d'acide inorganique comme par exemple l'acide chlorhydrique, l'acide sulfurique, ou l'acide bromhydrique ou d'un sel d'acide organique comme par exemple l'acide acétique, l'acide triflique, l'acide tartrique, l'acide oxalique, l'acide citrique, l'acide trifuoroacétique ou l'acide méthane sulfonique.

La présente description fournit, de plus, une composition pharmaceutique ou de diagnostic comprenant au moins un composé tel que précédemment défini dans un véhicule pharmaceutiquement acceptable.

Par « véhicule pharmaceutiquement acceptable », on entend dans le cadre de la présente invention un ou plusieurs adjuvants, excipients, tampons, diluants, et/ou autres auxiliaires pharmaceutiques habituels et connus de l'homme du métier.

La présente invention concerne l'utilisation d'un composé de formule (B) tel que précédemment défini, d'un sel de celui-ci ou d'une composition pharmaceutique ou de diagnostic comprenant au moins un tel composé dans un véhicule pharmaceutique acceptable pour des études d'imagerie PET appliquées au domaine de la leucémie lymphoïde chronique.

La présente invention concerne également un tel composé ou une telle composition pour utilisation dans l'évaluation du traitement de la leucémie lymphoïde chronique et la cartographie *in vivo* de cellules hématopoïétiques malignes.

Les exemples ci-dessous illustrent, à titre non limitatif, le procédé et les produits selon la présente invention et font référence aux figures annexées.

### BRÈVE DESCRIPTION DES DESSINS

La figure 1 présente les radio-chromatographies en couche mince (ou « radioCCM ») obtenues, à t=5 min, à partir du mélange réactionnel de fluoration mettant en oeuvre en tant que source de cations soit du K₂CO₃ (figure 1A), soit du K₂SO₄ (figure 1B).
La figure 2 présente l'évolution en fonction du temps de la composition du mélange réactionnel lors de l'étape de fluoration mettant en oeuvre en tant que source de cations du K₂SO₄.
La figure 3 présente l'évolution en fonction du temps de la composition du mélange réactionnel lors de l'étape de fluoration mettant en oeuvre en tant que source de cations du K₂CO₃.
La figure 4 présente la comparaison des pourcentages du produit partiellement déprotégé dans le milieu réactionnel en fonction du temps et de la nature du sel de potassium utilisé comme source de cations (K₂SO₄ ou K₂CO₃).

### EXPOSÉ DÉTAILLÉ DE MODES DE RÉALISATION PARTICULIERS

### MATÉRIEL ET MÉTHODES.

En général, tous les produits chimiques et solvants sont de qualité ACS (pour « Analytical grade chemical solvent ») ou de qualité HPLC (pour « High-Performance Liquid Chromatography » ou « chromatographie en phase liquide à haute performance ») et sont utilisés sans aucune autre purification sauf mention contraire.

Le dichlorométhane (CH₂Cl₂) a été distillé sur du P₂O₅. La pyridine a été séchée et distillée sur du KOH. L'acétonitrile, le DMF, le THF, le dioxane sont purifiés sur résine sur un appareil MBBraun-SPS-800. L'eau a été déionisée sur un appareil Millipore et filtrée avec un filtre 0,22 µm (Millipak). La fludarabine (2F-ARA-A) a été obtenue auprès de Sigma.

Les analyses HPLC ont été réalisées avec une pompe HPLC (modèle L-6200 Intelligent Pump, Merck), un détecteur UV Merck L-4250 (λ = 254 nm) en série avec un détecteur flux β⁺ de Novelec. Les chromatogrammes HPLC ont été enregistrés par un module interface/contrôle à double canal (Varian star 800) connecté à un PC avec le logiciel Galaxy (Varian).

Les chromatographies sur couche mince (TLC pour « Thin layer chromatographies ») ont été réalisées sur des plaques de silice (gel 60 F₂₅₄) et visualisées en utilisant une lampe UV (λ=254nm) ou par immersion dans un agent de coloration approprié (KMnO₄, Vanilline, Iode ou PMA) suivie par un chauffage doux. Les composés radioactifs ont été localisés sur les TLC en utilisant un imageur (Packard Instant Imager) connecté à un PC.

Les chromatographies Flash ont été réalisées sur des colonnes de gel de silice (SiO₂ 40-63 µm, Merck).

RMN (Résonance Magnétique Nucléaire) : les spectres RMN ¹H ont été enregistrés en utilisant un appareil Bruker à 250 ou 400 MHz (DPX 250 or DRX 400). Les déplacements chimiques δ sont indiqués en ppm en utilisant le TMS comme référence. Les constantes de couplage *J* sont données en Hertz (Hz). Les multiplicités sont indiquées par les abréviations suivantes : s = singulet, d= doublet, t = triplet, q = quartet, m = multiplet, bs = singulet large.

Les spectres RMN ¹³C {¹H} ont été enregistrés en utilisant un appareil Bruker à 62,9 ou 100,6 MHz. Les déplacements chimiques δ sont indiqués en ppm en utilisant le solvant deutérié comme référence. Les constantes de couplage *J* sont données en Hertz (Hz).

Les spectres RMN ¹⁹F ont été enregistrés en utilisant un appareil Bruker Advanced DRX 400 (376 MHz). Les déplacements chimiques δ sont indiqués en ppm en utilisant le CFCl₃ comme référence externe.

L'ion fluorure [¹⁸F]F⁻ dans l'eau a été obtenu à partir d'un cyclotron (IBA, Cyclone 18/9 RF) en utilisant un faisceau de protons sur de l'eau enrichie en [¹⁸O] (95%) (Cambridge Isotope Laboratories Inc.). L'ion fluorure [¹⁸F]F⁻ dans l'eau a été purifié sur une résine échangeuse d'ions QMA (ABX, Advanced biochemical compounds) éluée avec 500 µl de K₂CO₃ aqueux (1 mg/ml jusqu'à 5 mg/ml).

L'évaporation de l'eau a été réalisée par distillation azéotropique avec de l'acétonitrile à 110 °C sous un courant d'azote en utilisant un module agitateur/chauffant (Pierce).

Les mesures de radioactivité ont été faites en utilisant un calibrateur Capintec CRC-15 dose calibrator.

### EXEMPLE 1: PRÉPARATION DE [¹⁸F]FLUOROADENOSINE.

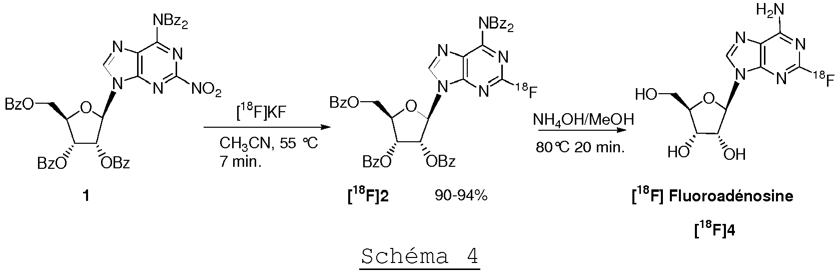

### CHIMIE.

L'adénosine pentabenzoylée et la 2-nitro-pentabenzoyladénosine **1** ont été préparées selon des méthodes déjà décrites (Article de Braendvang et al., 2006, Synthesis, 18, 2993 et demande internationale WO 2005/056571) et purifiées par chromatographie sur gel de silice.

Les 2-fluoro-adénosine **2** et **3** protégées ont été obtenues selon un protocole modifié (1,1 équivalent de Bu₄NF dans du CH₃CN au lieu d'un excès de réactif dans du DMF) permettant leur isolement dans des rendements élevés.

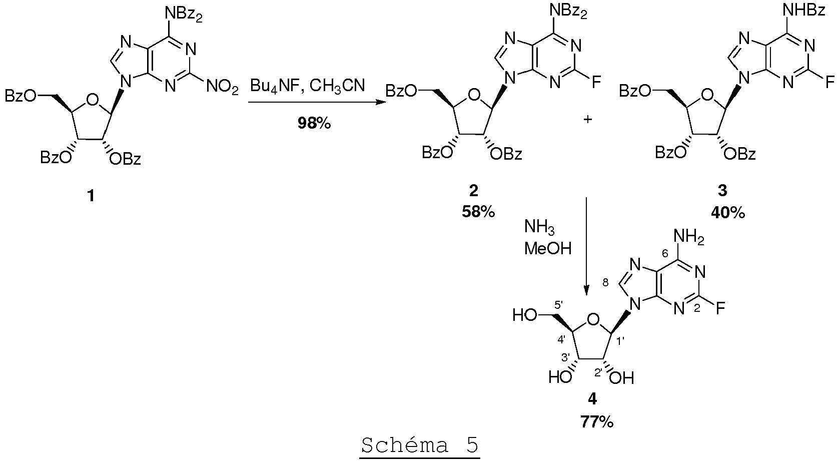

Du fluorure de tétrabutyl ammonium (TBAF) (1,3 eq, 600 µL, 0,6 mmol, 1 M dans du THF) a été ajouté goutte à goutte pendant 1 min à une suspension de 2-nitro-adénosine **1** (379 mg, 0,45 mmol) dans de l'acétonitrile sec (15 mL) à 0 °C. Le mélange a été agité pendant 20 min et la solution obtenue a été évaporée sous vide sans chauffage. Le produit a été purifié par chromatographie flash (CH₂Cl₂-acétone). Les composés **2** et **3** ont été isolés avec un rendement de 58% (211 mg) et 35% (130 mg) respectivement.

Une solution du composé **2** protégé (185 mg, 0,23 mmol) dans du MeOH (40 mL) a été saturée par un courant d'ammoniac gaz durant 15 min à 0 °C. Le mélange résultant a été agité pendant 14 h à température ambiante puis évaporé sous pression réduite et purifié par chromatographie flash sur gel de silice (AcOEt-MeOH 83:17) pour donner 51 mg (77%) de 2-fluoroadénosine **4.**

2-fluoro-6-*N,N*-dibenzoyl-9-(2',3',5'-tri-O-benzoyl-β-D-ribofuranosyl)-9H-purine **2.**
¹H NMR (400 MHz, CDCl₃) : δ = 4,82 (m, 3 H, H₄, H_{5'}); 6,17 (t, 1 H, H_{3'}, *J* = 5,4 Hz); 6,22 (t, 1 H, H_{2'}, *J* = 5.8 Hz); 6,46 (d, 1 H, H_{1'}, *J* = 5,3 Hz); 7,28-7,52 (m, 15 H, Ar); 7,85 (dd, 4H, *J* = 1,2 and 8,4 Hz; H_{Ar}); 7,95 (dd, 2H, *J =* 1,2 and 8,4 Hz, H_{Ar}); 8,02 (dd, 2H, *J* = 1,2 et 8,4 Hz, H_{Ar}); 8,12 (dd, 2H, *J* = 1,2 et 8.40, H_{Ar}); 8,20 (s, 1 H, H₈).
¹³C NMR (100,6 MHz, CDCl₃) : δ = 63,9 (C_{5'}); 71,7 (C_{4'}); 74,4 (C_{3'}); 81,4 **(C_{2'});** 87,0 (C_{1'}); 126,4 (d, *J*_{CF} = 5.0 Hz, C₅) ; 128,7; 128,9; 129,0; 129,1; 129,3; 129,5; 129,9; 130,1; 130,2; 130,3; 133,7; 133,9; 134,0; 134,2; 134,3; 143,7 (d, *J*_{CF} = 3,8 Hz, C₈); 154,3 (d, *J*_{CF} = 16,9 Hz, C₆); 154,9 (d, *J*_{CF} = 17,0 Hz, C₄) ; 158,3 (d, *J*_{CF} = 218,9Hz, C₂); 165,5 (C=O); 165,7 (C=O); 166,5 (C=O); 172,1 (2 x C=O).
¹⁹F NMR (376.5 MHz, CDCl₃) : δ = -49,1

2-fluoro-6-*N*-benzoyl-9-(2',3',5'-tri-O-benzoyl-β-D-ribofuranosyl)-9H-purine **3.**
(deux rotamères: rapport 80/20).
¹H NMR (400 MHz, CDCl₃) : δ = 4,74 (m, 3,6 H, H_{4'} H_{5'}); 6,12 (m, 2,4 H, H_{3'} H_{2'}); 6,39 (d, 0,8 H, H_{1'}, *J* = 5,5 Hz); 6,51 (d, 0,2 H, H_{1'}, *J* = 5,5 Hz); 7,28-7,51 (m, 15 H, H_{Ar}); 7,83-8,011 (m, 10 H, H_{Ar}); 8,08 (s, 0,8 H, **H₈);** 8,39 (s, 0,2 H, **H₈);** 8,94 (s, 0,8 H, NH); 9,14 (s, 0,2 H, NH).
¹⁹F NMR (376,5 MHz, CDCl₃) : δ = -47,6

2-fluoro-9-(β-D-ribofuranosyl)-9H-purine **4.**
¹H NMR (400 MHz, DMSO-D₆) : δ = 3,60 (m, 2 H, H_{5'}); 3,94 (m, 1 H, H_{4'}); 4,13 (m, 1 H, H_{3'}); 4,52 (m, 1 H, H_{2'}); 5,07 (t, 1 H, OH-5', *J* = 5,6 Hz); 5,20 (d, 1 H, OH-3', *J* = 4,7 Hz); 5,47 (d, 1 H, OH-2', *J* = 5,9 Hz); 5,79 (d, 1 H, H_{1'}, *J* = 5,9 Hz); 7,87 (bs, 2 H, NH₂); 8,35 (s, 1 H, H₈).
¹⁹F NMR (376, 5 MHz, DMSO-D₆) : δ = -52,5

### RADIOCHIMIE.

### Méthode A selon la présente invention

L'ion fluorure [¹⁸F]F⁻ dans l'eau a été adsorbé sur une résine échangeuse d'ions QMA (ABX) et élué avec du K₂CO₃ aqueux (500 µL, 1 mg/mL) et du Kryptofix (K₂₂₂, 15-25 mg) dans de l'acétonitrile (500 µL). L'eau a été évaporée sous un courant d'azote à 110 °C par distillation azéotropique en utilisant de l'acétonitrile (3 x 1 mL). Le précurseur **1** (5-5,5 mg dans 500-800 µL d'acétonitrile) a été ajouté au complexe [¹⁸F]KF sec. Un chauffage à 55-60 °C pendant 5 à 10 min (de préférence, 8 min) permet d'obtenir la [¹⁸F]fluoroadénosine **2** attendue dans un rendement radiochimique de plus de 90% (Chromatographie sur couche mince SiO₂ éluée avec CH₂Cl₂/acétone 95/5, Packard instant imager). La solution a été diluée avec 500 µL d'acétate d'éthyle (AcOEt) et adsorbée sur une cartouche de silice Sep-Pak (WATERS). L'élution avec 3-3,5 mL d'AcOEt suivie par 5 mL d'air sec et l'évaporation du solvant a permis d'obtenir la [¹⁸F]2-fluoroadénosine **2** protégée avec un rendement global de 86% (corrigé de la décroissance) à partir du [¹⁸F]KF.

La déprotection a été réalisée de façon conventionnelle par ajout de 500 µL de méthanol suivi de 500 µL d'ammoniac aqueux (29% dans de l'eau) et chauffage à 65-70 °C pendant 20 min. Après refroidissement, l'acide acétique (0,7 mL, 40% dans de l'eau ou 0,3 ml d'acide acétique pur) a été ajouté et la solution claire a été purifiée par une chromatographie HPLC semi-préparative sur une colonne µBondapak (eau/éthanol 97/3 5 mL/min) pour obtenir la [^{18F}]-fluoroadénosine **4** avec un rendement global de 50% (corrigé de la décroissance).

### Méthode B (ne faisant pas partie de la présente invention)

La méthode B est une alternative pour obtenir de façon sélective la fluoroadénosine [¹⁸F] mono hydrolysée **3.** La déprotection subséquente peut alors être réalisée en utilisant des conditions plus drastiques (80-85 °C) pour obtenir la [¹⁸F]-fluoroadénosine **4** et cette stratégie améliore l'étape de déprotection (90%).

A la [^{18F}]fluoroadénosine **2** brute dans l'acétonitrile (obtenue selon la méthode A) a été ajoutée de la 2-phényl-éthylamine (1 mg) dans 100 µL d'acétonitrile et la solution a été chauffée pendant 10 min à 60 °C. Une analyse en TLC et HPLC a montré une formation quantitative de l'intermédaire marqué mono-déprotégé qu'est le composé [¹⁸F] **3.** La solution a été diluée avec 500 µL d'AcOEt et adsorbée sur une cartouche de silice Sep-Pak (WATERS). L'élution avec 3,5 mL d'AcOEt suivie par 5 mL d'air sec et l'évaporation du solvant permet d'obtenir le composé [¹⁸F] **3** avec un rendement non optimisé de 60% (corrigé de la décroissance).

La déprotection finale a été réalisée par ajout de 500 µL de méthanol suivi par 400 µL d'ammoniac (29% dans de l'eau) et chauffage à 80-85 °C durant 20 min. Après refroidissemnt, l'acide acétique (0,7 mL, 40% dans de l'eau) a été ajouté et la solution claire a été purifiée par une chromatographie HPLC semi-préparative sur une colonne µBondapak (eau/éthanol 97/3, 5 mL/min) pour donner la 2-fluoroadénosine [¹⁸F] marquée **4.**

### EXEMPLE 2: PRÉPARATION DE [¹⁸F]FLUDARABINE.

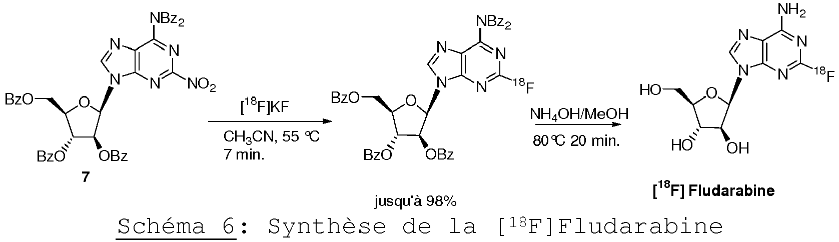

### CHIMIE

La synthèse du précurseur **7** peut être obtenue par protection et nitration de la 9(β-D-arabinofuranosyl)-9H-purine selon les protocoles décrits pour son analogue ribose (Article de Braendvang et al., 2006, Synthesis, 18, 2993 et demande internationale WO 2005/056571).

De façon alternative, le composé **7** peut également être obtenu en partant de l'adénosine ou de la guanosine (schéma 6) (Article de Gimisis et al., 1998, Tetrahedron, 54, 573 et demande internationale WO 9412514).

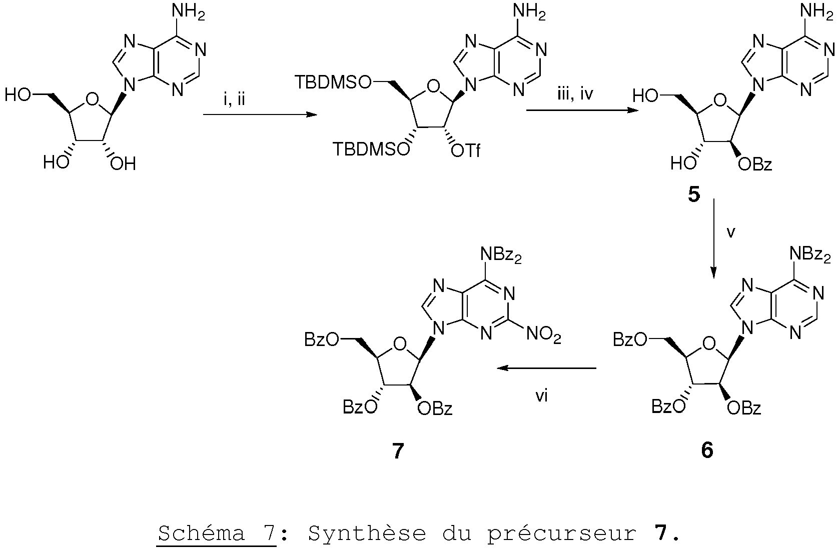

Les réactifs et rendements des différentes étapes de la synthèse du précurseur **7** (schéma 7) sont :
i: TBDMSCl, DABCO, AgNO₃, THF, 53%.
ii: Tf₂O, DMAP, DIPEA, Py. 67%.
iii: PhCOOK, DMSO, 96%.
iv: Bu₄NF, THF, 81%.
v: PhCOCl, Py, 95%.
vi: TBAN, TFAA, CH₂Cl₂, 45%.

Synthèse de la 6-N,N-dibenzoyl-9-(2',3',5'-tri-O-benzoyl-β-D-arabinofuranosyl)-9H-purine **6.**

Le chlorure de benzoyle (750 µL, 6,49 mmol) a été ajouté à une solution du composé **5** (181 mg, 0,82 mmol) dans de la pyridine anhydre (7 mL) et le mélange a été chauffé à reflux pendant 6 h. Après refroidissement et extraction (CH₂Cl₂), la phase organique a été lavée avec du NaHCO₃ aqueux saturé (10 mL) et de la saumure (2 x 10 mL), et séchée sur du Na₂SO₄. La filtration et l'évaporation sous pression réduite ont produit le produit brut sous forme d'un solide jaune pâle, qui a été purifié par chromatographie flash (CH₂Cl₂ : acétone, 95:5) pour donner 606 mg (96%) de **6.**
¹H NMR (250 MHz, CDCl₃) : δ = 4, 73 (m, 3 H, H_{4'} and H_{5'}); 5,91 (m, 2 H, H_{3'} and H_{2'}); 6,48 (d, 1 H, H_{1'}, *J* = 4,2 Hz); 7,20-7,39 (m, 15 H, H_{Ar}) ; 7,68-7,72 (m, 5 H, H_{Ar}) ; 7,96-8,00 (m, 5 H, H_{Ar}) ; 8,38 (s, 1 H, H₂); 8,58 (s, 1 H, H₈).

6-*N,N*-dibenzoyl-9-(2',3',5'-tri-O-benzoyl-β-D-arabinofuranosyl)-2-nitro-9H-purine **7.**

Un mélange nitré a été préparé par ajout d'anhydride 2,2,2-trifluoroacétique (160 µL, 1,15 mmol) pendant 2 min à une solution de nitrate de tétrabutylammonium (351 mg, 1,15 mmol) dans du chlorure de méthylène sec (15 mL) à 0 °C. Après 45 min, la solution a été ajoutée à **6** (606 mg, 0,77 mmol) dans du chlorure de méthylène sec (15 mL) à 0 °C. Après 14 h à température ambiante (et protégé de la lumière), le mélange réactionnel a été versé dans un mélange froid d'eau (30 mL), de NaHCO₃ aqueux saturé (20 mL) et de CH₂Cl₂-Et₂O (1:2, 20 mL). La couche aqueuse a été extraite avec du CH₂Cl₂-Et₂O (1:2, 2 x 20 mL). Les extraits organiques combinés ont été lavés avec de la saumure (2 x 20 mL), séchés (Na₂SO₄), et évaporés sous vide (sans chauffage au-delà de 40 °C). Le produit a été purifié par chromatographie flash (CH₂Cl₂-acétone 95:5) pour produire 201 mg (45%) de **7.**
¹H NMR (250 MHz, CDCl₃) : δ = 4,79 (m, 3 H, H_{4'} H_{5'}); 5,86 (m, 2 H, H_{3'} H_{2'}) ; 6,83 (d, 1 H, H_{1'}, *J* = 5,1 Hz); 7,21-7,47 (m, 15 H, H_{Ar}); 7,66-7,73 (m, 6 H, H_{Ar}); 7,95 (dd, 2 H, *J =* 1,4 and 7,8 Hz, H_{Ar}); 8,05 (dd, 2H, *J* = 1,4 and 7,8 Hz, H_{Ar}); 8,52 (s, 1 H, H₈).
¹³C NMR (62,9 MHz, CDCl₃) : δ = 63,5 (C_{5'}); 76,3 (C_{2'}); 77,1 (C_{3'}); 81,6 (C_{4'}); 84,7 (C_{1'}); 127,8 (C₅); 128,6; 128,9; 129,1; 129,2; 129,3; 129,5; 129,6; 129,8; 130,1; 130,2; 130,5; 133,7; 133,8; 133,9; 134,5; 134,6; 147,7 (C₈); 153,0 (C₆); 153,1 (C₂); 154,0 (C₄); 165,1 (C=O); 165,8 (C=O); 166,6 (C=O); 171,8 (2 x C=O).

### RADIOCHIMIE

### [18F]-FLUDARABINE:

En utilisant 4,8 à 5,5 mg de 6-*N,N*-dibenzoyl-9-(2',3',5'-tri-O-benzoyl-β-D-arabinofuranosyl)-2-nitro-9H-purine **7** et les procédés (A ou B) précédemment décrits, la [¹⁸F]-fludarabine a été obtenue avec un rendement radiochimique global de 63% (corrigé de la décroissance) en environ de 85 min après la purification par HPLC semi-préparative (Eau/Ethanol 97/3 5mL/min).

### EXEMPLE 3: ÉVOLUTION DU MÉLANGE RÉACTIONNEL DE FLUORATION EN FONCTION DU SEL DE POTASSIUM UTILISÉ COMME SOURCE DE CATIONS.

La réaction de fluoration est effectuée dans le DMSO à 140°C en présence de sel de potassium (K₂CO₃, 0,5-1 mg ou K₂SO₄ 3-5 mg) et de kryptofix K2.2.2 (20 mg) en utilisant 5 mg de précurseur (2-nitro purine). Les produits réactionnels obtenus présentent la structure suivante :

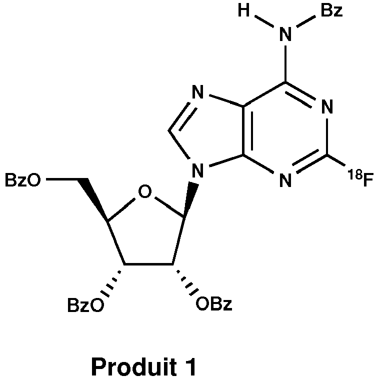

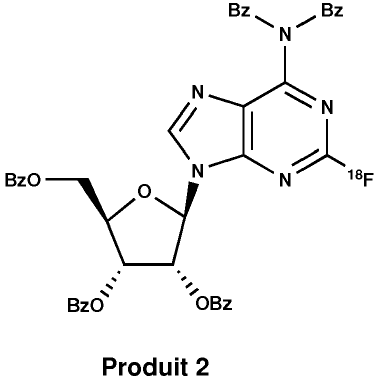

L'analyse du mélange réactionnel est réalisée par radio-CCM (SiO₂, éluent CH₂Cl₂/Acétone 95/5) à partir d'un prélèvement de 15-25 µL effectué toutes les 5 minutes.

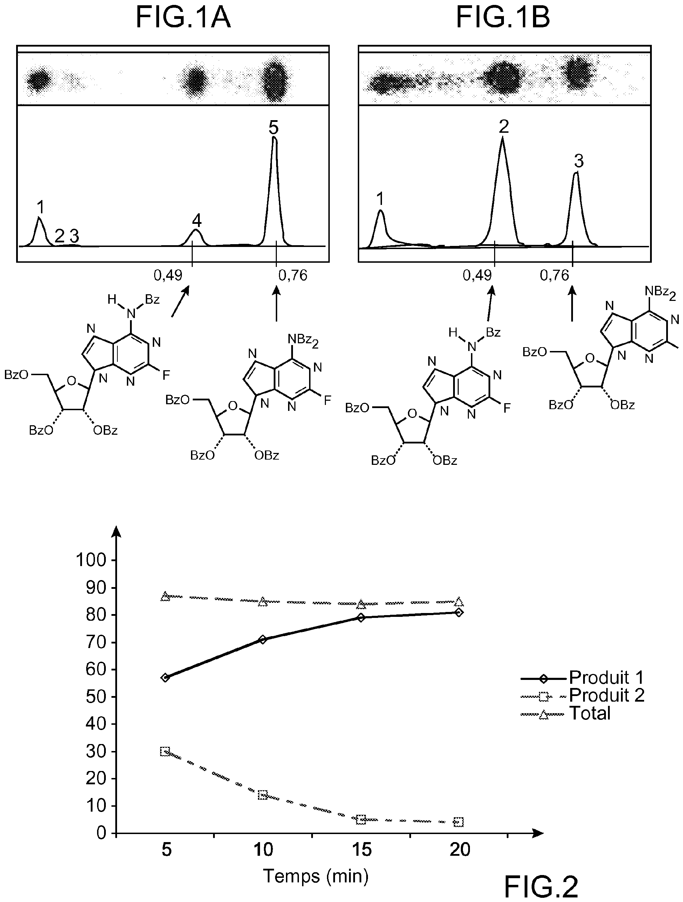

Les produits radioactifs sont identifiés par co-élution avec des références non radioactives (produit **1** Rf = 0,49 ; produit **2** Rf = 0,75 ; cf Figure 1).

La Figure 2 montre qu'en présence de K₂SO₄, le produit **1** (partiellement déprotégé) est formé de façon majoritaire lors de la réaction de fluoration. La composition du mélange évolue en fonction du temps et le produit **1** devient largement majoritaire au bout de 15 minutes au détriment du produit **2** (déprotection progressive du produit **2** dans ces conditions réactionnelles).

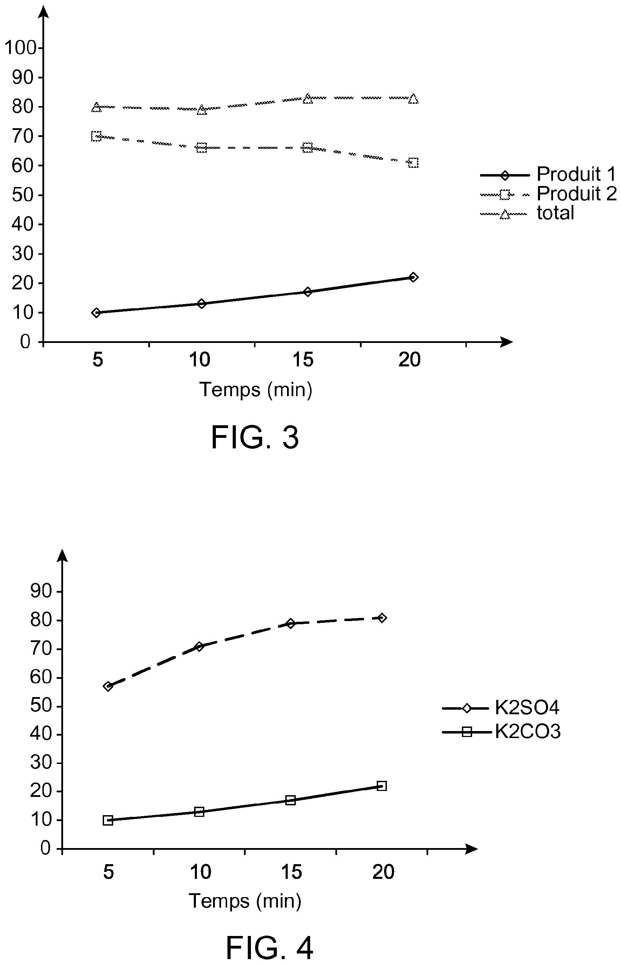

Lorsque la réaction de fluoration est réalisée en présence de K₂CO₃, le produit **2** (entièrement protégé) est formé de façon majoritaire et sa proportion demeure stable au cours du temps (61 à 70%), comme présenté à la Figure 3.

La Figure 4 permet d'observer l'influence de la nature du sel de potassium (K₂SO₄ versus K₂CO₃) sur la formation du produit **1.** L'utilisation du K₂SO₄ permet d'obtenir le produit **1** de façon majoritaire et après 20 minutes de réaction le produit **1** représente 95% (ratio **1/2** = 81/4) des produits de fluoration.

### RÉFÉRENCES BIBLIOGRAPHIQUES

Article de Jacobson K.A. et Gao Z.-G. Nat. Rev. Drug Discovery 2006, 5, 247 ;
Demande internationale WO 03/099342 ;.
Article de Robins M.J. et Uznanski B., 1981, Can. J. Chem. 59, 2608 ;
Article de Horti et al., 2006, J. Labelled Compd. Radiopharm., 49, 811 ;
Demande internationale WO 2005/044312 ;
Article de Hocek et al., 2005, Eur. J. Org. Chem. 14, 3026 ;
Demande de brevet EP 1 956 013 ;
Article de Irie et al., 1982, Int. J. Appl. Radiat. Isot. 33(6), 445 ;
Demande internationale WO 2005/056571 ;
Article de Wanner et al., Med. Chem. Lett. 10, 2000b, 2141-2144 ;
Article de Wanner et Koomen, J. Chem. Soc., Perkin Trans 1, 2001, 1908-1915 ;
Article de Degati et al., Tetrahedron lett., 41, 2000, 1291-1295 ;
Article de Nowak et al., J. Org Chem. 70, 2005, 7455-7458 ;
Article de Ishidoet al., J. Chem. Soc., Perkin Trans. 1, 1977, 657-660 ;
Article de Braendvang et al., 2006, Synthesis, 18, 2993 ;
Article de Gimisis et al., 1998, Tetrahedron, 54, 573 ;
Demande internationale WO 94/12514.

## Revendications

1. Procédé de préparation d'un dérivé de 2-fluoro-purine marqué avec le radioisotope ¹⁸F de formule 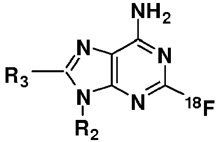 dans laquelle
R₂ représente H, un groupe alkyle éventuellement substitué, un groupe aryle éventuellement substitué, un groupe furanose éventuellement substitué ou un groupe pyranose éventuellement substitué,
R₃ représente H, un groupe alkyle éventuellement substitué, un groupe aryle éventuellement substitué, un halogène, un groupe -OR₈ ou un groupe -SR₈, avec R₈ représentant H, un groupe alkyle éventuellement substitué ou un groupe aryle éventuellement substitué,
ledit procédé comprenant les étapes successives suivantes consistant à :
a) faire réagir un dérivé de 2-nitro-purine protégé de formule (Ic) 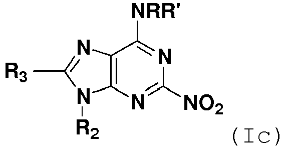 dans laquelle
R et R' sont des groupes protecteurs, identiques ou différents, choisis parmi un groupe alkyle éventuellement substitué, un groupe acyle éventuellement substitué, un groupe aryle éventuellement substitué, un groupe benzyle éventuellement substitué, un groupe benzoyle éventuellement substitué, un groupe électroattracteur, un groupe sulfinyle éventuellement substitué, un groupe sulfonyle éventuellement substitué, un groupe trityle, un groupe silyle, un groupe tertiobutoxycarbonyle (BOC), un groupe fluorénylméthoxy carbonyle (FMOC) et un dérivé d'imide ou un dérivé d'imine, et
R₂ et R₃ sont tels que précédemment définis, avec une source d'ions fluorure F- marqués au [¹⁸F] dans un solvant qu'est l'acétonitrile, à une température de 55 à 60°C et pendant une durée comprise entre 1 et 30 min, pour obtenir un dérivé de 2-[¹⁸F]fluoro-purine protégé et éventuellement un dérivé de 2-[¹⁸F]fluoro-purine partiellement déprotégé,
ledit dérivé de 2-[¹⁸F]fluoro-purine protégé étant de formule (Id) 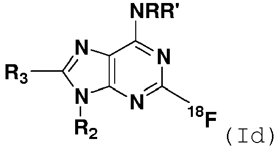
avec R₂, R₃, R et R' tels que précédemment définis,
ledit dérivé de 2-[¹⁸F]fluoro-purine partiellement déprotégé étant de formule (Ie) 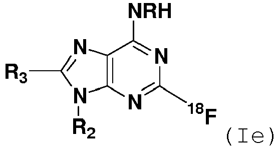
avec R₂, R₃ et R tels que précédemment définis,
b) déprotéger ledit dérivé de 2-[¹⁸F]fluoro-purine protégé et ledit dérivé de 2-[¹⁸F]fluoro-purine partiellement déprotégé éventuellement obtenu pour obtenir le dérivé de 2-[¹⁸F]fluoro-purine en faisant réagir ledit dérivé de 2-[¹⁸F]fluoro-purine protégé et ledit dérivé de 2-[¹⁸F]fluoro-purine partiellement déprotégé éventuellement obtenu avec alcool, suivi d'ammoniac aqueux puis en chauffant à une température comprise entre 50 et 90°C pendant une durée comprise entre 5 et 45 min.

2. Procédé de préparation selon la revendication 1, **caractérisé en ce que** la source d'ions fluorure marqués au ¹⁸F mis en oeuvre à l'étape de fluoration comprend lesdits ions fluorure et un contre-ion.

3. Procédé de préparation selon la revendication 1, **caractérisé en ce que** ladite étape (a) consiste à faire réagir un dérivé de 2-nitro-purine protégé de formule (Ic) tel que défini à la revendication 1 avec une source d'ions fluorure F-marqués au [¹⁸F], avec du K₂SO₄ comme contre-ion, pour préparer un dérivé de 2-[¹⁸F]fluoro-purine partiellement déprotégé de formule (Ie) tel que défini à la revendication 1.

4. Utilisation d'un composé de formule (B) 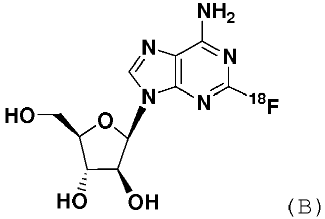 ou d'un sel de celui-ci
ou d'une composition pharmaceutique ou de diagnostic, comprenant au moins un composé de formule (B) dans un véhicule pharmaceutiquement acceptable
pour des études d'imagerie PET appliquées au domaine de la leucémie lymphoïde chronique.

5. Composé ou composition tel(le) que défini(e) à la revendication 4 pour utilisation dans l'évaluation du traitement de la leucémie lymphoïde chronique et la cartographie *in vivo* de cellules hématopoïétiques malignes.

## Patentansprüche

1. Verfahren zur Herstellung eines Derivats von 2-Fluor-Purin, das mit dem Radioisotop ¹⁸F der Formel 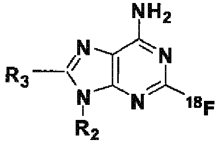 markiert ist, wobei
R₂ H repräsentiert, eine gegebenenfalls substituierte Alkylgruppe, eine gegebenenfalls substituierte Arylgruppe, eine gegebenenfalls substituierte Furanosegruppe, oder eine gegebenenfalls substituierte Pyranosegruppe,
R₃ H repräsentiert, eine gegebenenfalls substituierte Alkylgruppe, eine gegebenenfalls substituierte Arylgruppe, ein Halogen, eine Gruppe -OR₈ oder eine Gruppe -SR₈, wobei R₈ H repräsentiert, eine gegebenenfalls substituierte Alkylgruppe oder eine gegebenenfalls substituierte Arylgruppe,
wobei das Verfahren die folgenden aufeinanderfolgenden Schritte umfasst:
a) Reagierenlassen eines geschützten Derivats von 2-Nitro-Purin der Formel (Ic) 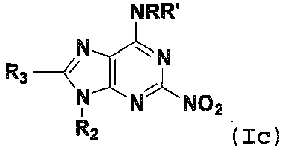 wobei
R und R' identische oder unterschiedliche Schutzgruppen sind, ausgewählt aus einer gegebenenfalls substituierten Alkylgruppe, einer gegebenenfalls substituierten Acylgruppe, einer gegebenenfalls substituierten Arylgruppe, einer gegebenenfalls substituierten Benzylgruppe, einer gegebenenfalls substituierten Benzoylgruppe, einer elektronenziehenden Gruppe, einer gegebenenfalls substituierten Sulfinylgruppe, einer gegebenenfalls substituierten Sulfonylgruppe, einer Tritylgruppe, einer Silylgruppe, einer Tertiobutoxycarbonylgruppe (BOC), einer Fluorenylmethoxycarbonylgruppe (FMOC) und einem Derivat von Imid oder einem Derivat von Imin, und
R₂ und R₃ wie vorstehend definiert sind,
mit einer Quelle von Fluoridionen F-, die mit [¹⁸F] markiert sind, in einem Lösungsmittel, das Acetonitril ist, bei einer Temperatur von 55 bis 60°C und während einer Dauer, die zwischen 1 und 30 Minuten enthalten ist, um ein geschütztes Derivat von 2-[^{1β}F]Fluor-Purin und gegenbenenfalls ein partiell entschütztes Derivat von 2-[¹⁸F]Fluor-Purin zu erhalten,
wobei das geschützte Derivat von 2-[¹⁸F]Fluor-Purin die Formel (Id) hat 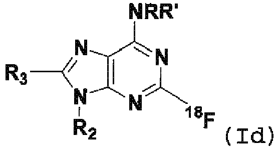
mit R₂, R₃, R und R' wie vorstehend definiert,
wobei das teilweise entschützte Derivat von 2-[¹⁸F]Fluor-Purin die Formel (Ie) hat 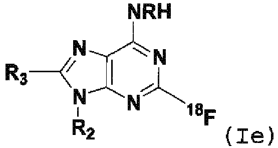
mit R₂, R₃ und R wie vorstehend definiert,
b) Entschützen des geschützten Derivats von 2-[¹⁸F]Fluor-Purin und des gegebenenfalls erhaltenen teilweise entschützten Derivats von 2-[¹⁸F] Fluor-Purin, um das Derivat von 2-[¹⁸F]Fluor-Purin zu erhalten durch Reagieren lassen des geschützten Derivats von 2-[¹⁸F]Fluor-Purin und des gegebenenfalls erhaltenen teilweise entschützten Derivats von 2-[¹⁸F]Fluor-Purin mit Alkohol, gefolgt von wässrigem Ammoniak, und dann Erhitzen auf eine Temperatur, die zwischen 50 und 90°C enthalten ist, während einer Dauer, die zwischen 5 und 45 Minuten enthalten ist.

2. Herstellungsverfahren nach Anspruch 1, **dadurch gekennzeichnet, dass** die im Fluorierungsschritt verwendete Quelle von Fluoridionen, die mit **¹⁸F** markiert sind, die Fluoridionen und ein Gegenion umfasst.

3. Herstellungsverfahren nach Anspruch 1, **dadurch gekennzeichnet, dass** der Schritt (a) darin besteht, ein geschütztes Derivat von 2-Nitro-Purin der Formel (Ic) wie in Anspruch 1 definiert mit einer Quelle von Fluoridionen F-, die mit [¹⁸F] markiert sind, reagieren zu lassen, mit K₂SO₄ als Gegenion, um ein partiell entschütztes Derivat von 2-[¹⁸F]Fluor-Purin der Formel (Ie) wie in Anspruch 1 definiert herzustellen.

4. Verwendung einer Verbindung der Formel (B) 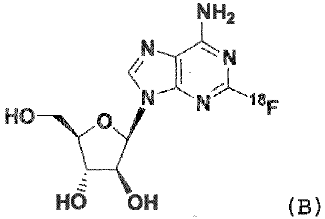 oder eines Salzes derselben
oder einer pharmazeutischen oder diagnostischen Verbindung, die wenigstens eine Verbindung der Formel (B) umfasst, in einem pharmazeutisch akzeptablen Träger
für PET-Bildgebungsstudien, die auf das Gebiet der chronischen lymphatischen Leukämie angewandt werden.

5. Verbindung oder Zusammensetzung wie in Anspruch 4 definiert für die Verwendung in der Bewertung der Behandlung der chronischen lymphatischen Leukämie und der In-Vivo-Kartographie von malignen blutbildenden Zellen.

## Claims

1. Method for preparing a 2-fluoropurine derivative marked with the ¹⁸F radioisotope of formula 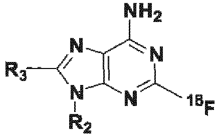 in which
R₂ represents H, an optionally substituted alkyl group, an optionally substituted aryl group, an optionally substituted furanose group, or an optionally substituted pyranose group;
R₃ represents H, an optionally substituted alkyl group, an optionally substituted aryl group, a halogen, a -OR₈ group or a -SRs group, with R₈ representing H, an optionally substituted alkyl group or an optionally substituted aryl group,
said method comprising the following successive steps, involving:
a) reacting a protected 2-nitropurine derivative of a formula (Ic) 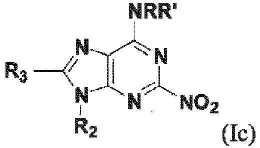 in which
R and R' are identical or different protecting groups chosen from among an optionally substituted alkyl group, an optionally substituted acyl group, an optionally substituted aryl group, an optionally substituted benzyl group, an optionally substituted benzoyl group, an electroattractive group, an optionally substituted sulfinyl group, an optionally substituted sulfonyl group, a trityl group, a silyl group, a tert-butoxycarbonyl (BOC) group, a fluorenylmethoxy carbonyl (FMOC) group, and an imide derivative or an imine derivative, and
R₂ and R₃ are as previously defined,
with a source of [¹⁸F] marked fluoride ions F⁻ in a solvent which is acetonitrile, at a temperature between 55 and 60°C and for between 1 and 30 min, to obtain a protected 2-[¹⁸F]fluoropurine derivative and optionally a partially unprotected 2-[¹⁸F]fluoropurine derivative,
said protected 2-[¹⁸F] fluoropurine derivative being of formula (Id) 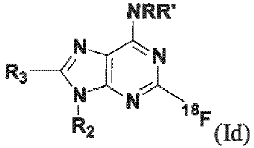
with R₂, R₃, R and R' being such as previously defined,
said partially deprotected 2-[¹⁸F]fluoropurine derivative being of formula (Ie) 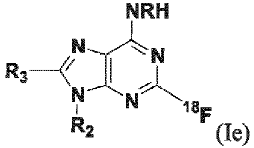
with R₂, R₃ and R being such as previously defined,
b) unprotecting said protected 2-[¹⁸F] fluoropurine derivative and said partially unprotected 2-[¹⁸F] fluoropurine derivative optionally obtained for achieving the 2-[¹⁸F] fluoropurine derivative, by reacting the protected 2-[¹⁸F] fluoropurine derivative and the optionally present partially unprotected 2-[¹⁸F] fluoropurine derivative with an alcohol, followed by aqueous ammonia and then by heating at a temperature between 50 and 90°C for between 5 and 45 min.

2. Preparation method according to claim 1, wherein the source of [¹⁸F] marked fluoride ions implemented during the fluorination step comprises said fluoride ions and a counter-ion.

3. Preparation method according to claim 1, wherein said step (a) consists in reacting a protected 2-nitropurine derivative of a formula (Ic) such as defined in claim 1 with a source of [¹⁸F] marked fluoride ions F⁻, with K₂SO₄ as counter-ion, to prepare a partially unprotected 2-[¹⁸F] fluoropurine derivative of formula (Ie) as defined in claim 1.

4. Use of a compound of formula (B) 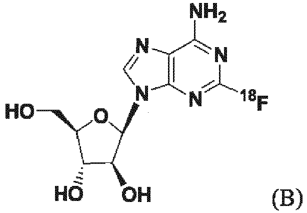 or a salt thereof
or a pharmaceutical or diagnostic composition, comprising at least one compound of formula (B) in an acceptable pharmaceutical vehicle,
for PET imaging studies applied to the fields of chronic lymphoid leukemia.

5. Compound or composition as defined in claim 4 for use in the treatment evaluation of the chronic lymphoid leukemia and the *in vivo* mapping of malignant hematopoietic cells.
